# DEMANDE DE BREVET EUROPEEN

(11) **EP 1 970 017 A1**
(43) Date de publication de la demande: **17.09.2008**
(21) Numéro de dépôt: 08300144.6
(22) Date de dépôt: 14.03.2008
(51) Int. Cl.: A61B 17/28

(54) **Ensemble a poignees, notamment pour instrument chirurgical endoscopique**

(30) Priorité: 14.03.2007 FR 0701851
(71) Demandeur: Landanger, 52000 Chaumont (FR)
(72) Inventeur: Landanger, Benoît, 52360, Changey (FR); Chaignaud, Jean, 33640, Beautiran (FR)
(74) Mandataire: Oudin, Stéphane

(57) **Abrégé**

Ensemble à poignées, notamment pour instrument chirurgical endoscopique, comprenant un corps tubulaire (12) destiné à recevoir une extrémité d'une tringlerie (8) mobile axialement dans le corps tubulaire, un premier élément de poignée (16) monté fixe au corps tubulaire, un deuxième élément de poignée (18) monté pivotant sur le corps tubulaire, et un moyen de couplage pour convertir un mouvement de pivotement du deuxième élément de poignée en un mouvement de translation axial de la tringlerie.

Le moyen de couplage comprend une pièce de couplage (22) montée pivotante sur le corps tubulaire (12), et reliée par une bielle (26) au deuxième élément de poignée (18), celle-ci comprenant un chemin pour guider un mouvement axial de l'extrémité de la tringlerie, en configuration opérationnelle, au moins une partie du chemin et l'extrémité de la tringlerie étant configurés pour qu'un frottement suffisant existe et bloque les éléments de poignée en position lorsque ces éléments de poignée ne sont pas actionnés.

## Description

La présente invention concerne un ensemble à poignées, notamment pour instrument chirurgical endoscopique, et un instrument chirurgical endoscopique comportant un tel ensemble à poignées.

Les instruments chirurgicaux endoscopiques comprennent un tube ayant une extrémité distale et une extrémité proximale, un ensemble à poignées étant monté sur l'extrémité proximale et un organe de travail étant monté sur l'extrémité distale. L'ensemble à poignées et l'organe de travail sont reliés par une tringlerie apte à coulisser dans le tube de sorte que l'actionnement des poignées provoque un mouvement de l'organe de travail.

L'organe de travail est typiquement de type pince ou organe de coupe et comprend généralement deux mors montés pivotant l'un par rapport à l'autre.

Pendant la phase où l'opérateur (chirurgien ou autre) déplace l'instrument pour le positionner correctement au niveau de la partie de tissu du patient à traiter, il est important que l'organe de travail ne soit pas actionné pour ne pas atteindre un tissu qui n'est pas à traiter. Il est donc nécessaire de pouvoir bloquer dans une position donnée l'organe de travail tant que l'instrument n'est pas correctement positionné.

Les systèmes de blocage connus dans l'état de la technique sont généralement de type à crémaillère crantée, fixe ou mobile, pour solidariser les deux poignées. De tels dispositifs de blocage requièrent un geste particulier du chirurgien pour le blocage ou le déblocage de l'instrument. Ce geste est délicat car la manoeuvre de blocage/déblocage est effectuée par le petit doigt ou un autre doigt, dont la mobilité est affectée par le fait que le pouce et l'index sont déjà engagés dans les anneaux des deux poignées.

En outre, les systèmes de blocage à crémaillère augmentent de manière considérable l'encombrement des instruments. Ils sont aussi susceptibles de s'accrocher à d'autres instruments et/ou à l'opérateur ou au patient, ce qui peut induire un déblocage intempestif et par suite un actionnement incontrôlé de l'organe de travail.

Par ailleurs, le brevet américain US5.468.250 propose une variante d'exécution d'un instrument chirurgical endoscopique faisant intervenir un système de blocage par friction des poignées d'actionnement.

Le mécanisme à friction est entièrement disposé dans les poignées. Il se compose d'un chemin de guidage aménagé dans l'élément de poignée monté pivotant sur le corps de l'instrument, à proximité de l'extrémité distale dudit élément. Une bille de guidage reliée à l'extrémité proximale du câble d'actionnement de l'outil chirurgical est engagée dans ce chemin. Le coefficient de friction entre le chemin et l'extrémité du câble est tel qu'il assure le maintien en position des poignées dès qu'elles ne sont pas actionnées. Le chemin a une forme d'arc circulaire réalisé de telle manière qu'un pivotement de 18° des poignées entraîne un déplacement de 0,33 cm du câble d'actionnement.

Ce dispositif, s'il résout les problèmes d'encombrement des systèmes à crémaillère, présente toutefois un manque de précision et de finesse dans le contrôle de l'actionnement de l'outil chirurgical, qui nuit fortement à la qualité du travail du chirurgien.

Le but de la présente invention est de pallier les inconvénients des instruments connus.

A cette fin, l'invention a pour objet un ensemble à poignées, notamment pour instrument chirurgical endoscopique, comprenant un corps tubulaire destiné à recevoir une extrémité d'une tringlerie mobile axialement dans ledit corps tubulaire, un premier élément de poignée monté fixe sur ledit corps tubulaire, un deuxième élément de poignée monté de manière pivotante sur ledit corps tubulaire, et un moyen de couplage pour convertir un mouvement de pivotement du deuxième élément de poignée en un mouvement de translation axial de ladite tringlerie, remarquable en ce que ledit moyen de couplage comprend une pièce de couplage montée pivotante sur ledit corps tubulaire et reliée par une bielle au deuxième élément de poignée, ladite pièce de couplage comprenant un chemin pour guider un mouvement axial de l'extrémité de la tringlerie, en configuration opérationnelle, au moins une partie dudit chemin et l'extrémité de la tringlerie étant configurés pour qu'un frottement suffisant existe et bloque les éléments de poignée en position lorsque ces éléments de poignée ne sont pas actionnés.

Ainsi, selon l'invention, le blocage ou le déblocage de l'instrument ne nécessite pas de la part de l'opérateur une action particulière, autre que celle d'actionner l'ensemble à poignées, c'est-à-dire le geste naturel de rapprocher ou d'éloigner les éléments de poignée l'un de l'autre.

En outre, et avantageusement, l'ensemble effecteur, constitué par la bielle et la pièce intermédiaire dans laquelle est ménagée le chemin de guidage, assure à l'utilisateur un contrôle fin et précis du déplacement de la tringlerie en fonction de l'angle d'ouverture des poignées.

De manière préférée, le chemin et l'extrémité de la tringlerie sont configurés pour qu'un frottement bloquant les éléments de poignées existe sur l'ensemble dudit chemin.

Selon un mode de réalisation avantageux, ledit chemin est formé d'une rainure ou d'une fente.

De manière préférée, au moins une partie du chemin a un profil en spirale par rapport au pivot de la pièce de couplage.

Par profil en spirale en entend que lorsqu'on se déplace le long du chemin la distance au pivot varie (augmente ou diminue) de manière régulière.

L'invention a également pour objet un instrument chirurgical endoscopique comprenant :
- un tube ayant une extrémité proximale et une extrémité distale, une tringlerie étant disposée dans ledit tube,
- un organe de travail disposé à l'extrémité distale du tube et étant fixé à une extrémité de la tringlerie, et
- un ensemble à poignées selon l'invention monté à l'extrémité proximale du tube.

De manière avantageuse, l'extrémité proximale de la tringlerie est équipé d'une goupille agencée pour se mouvoir sur le chemin du moyen de couplage.

L'invention sera mieux comprise à la lecture des modes de réalisation qui suivent, donnés à titre illustratif mais non limitatif, en référence aux dessins annexés, sur lesquels :
- la figure 1 illustre un mode de réalisation d'une pince endoscopique selon l'invention,
- la figure 2 est une vue partielle de l'ensemble à poignées de la pince endoscopique de la figure 1, et
- les figures 3 et 4 illustrent schématiquement l'influence du profil du chemin sur la condition de blocage,
- les figures 5 et 6 sont des vues schématiques d'une variante d'exécution d'une pince endoscopique de l'invention, respectivement en position mors fermés et mors ouverts.

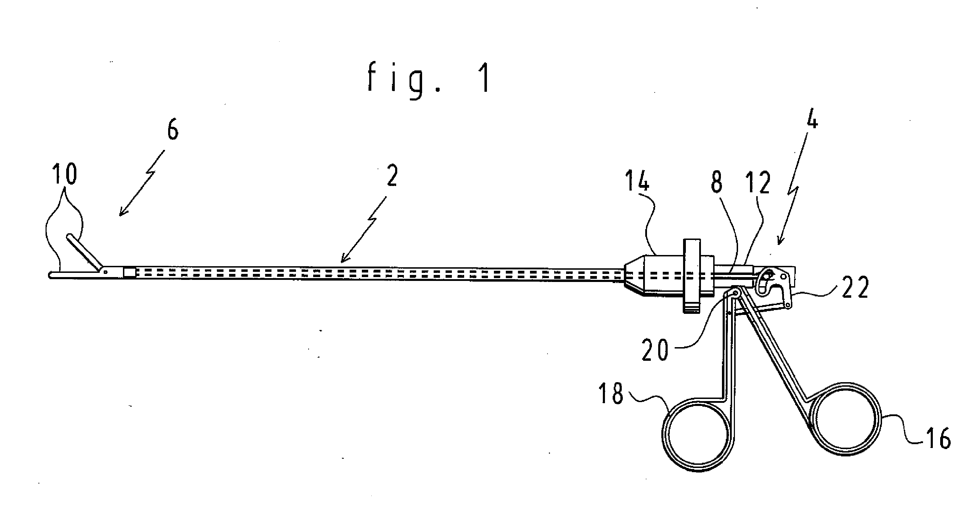

On a représenté sur la figure 1 un instrument chirurgical endoscopique selon l'invention. Cet instrument se compose de trois parties : un tube 2 ayant une extrémité proximale (proche de l'opérateur) et une extrémité distale (proche de la zone de travail), un ensemble à poignées 4 monté sur l'extrémité proximale et un organe de travail 6.

De manière classique, une tringlerie, dont une extrémité 8 est visible, est disposée à coulissement axial dans le tube 2 et relie mécaniquement l'ensemble à poignées 4 et l'organe de travail 6. Dans l'exemple représenté, l'organe de travail comprend deux mors 10. Ceux-ci sont montés pivotants l'un par rapport à l'autre. La tringlerie peut être notamment un câble ou une tige.

L'ensemble à poignées comprend un corps tubulaire 12 en prolongement de l'axe du tube 2. Le corps tubulaire 12 et le tube 2 sont solidarisés par une pièce de liaison 14.

L'ensemble à poignées 4 comprend en outre un premier élément de poignée 16 monté fixe au corps tubulaire 12 et un deuxième élément de poignée 18 monté pivotant par rapport au premier élément de poignée 16 au moyen d'un pivot 20.

L'ensemble à poignées 4 comprend également un moyen de couplage pour convertir le mouvement de pivotement du deuxième élément de poignée 18 en un mouvement de translation axial de la tringlerie 8.

Ce moyen de couplage comprend une pièce de couplage 22 montée sur un pivot 24 disposé selon un diamètre du corps tubulaire 12 et parallèlement au pivot 20, et une bielle 26 reliant la pièce de couplage 22 au deuxième élément de poignée 18. Dans le cas représenté, le premier élément de poignée 16 comporte une lumière 28 pour le libre passage de la bielle 26.

La pièce de couplage 22 comprend un chemin 30, sous forme de rainure ou de lumière, destiné à guider une goupille 32 fixée à l'extrémité de la tringlerie 8. Deux lumières 33, dont une seule est représentée, sont pratiquées dans le corps tubulaire 12 pour recevoir les extrémités de la goupille 32 et assurer ainsi son maintien sur l'axe du corps tubulaire 12.

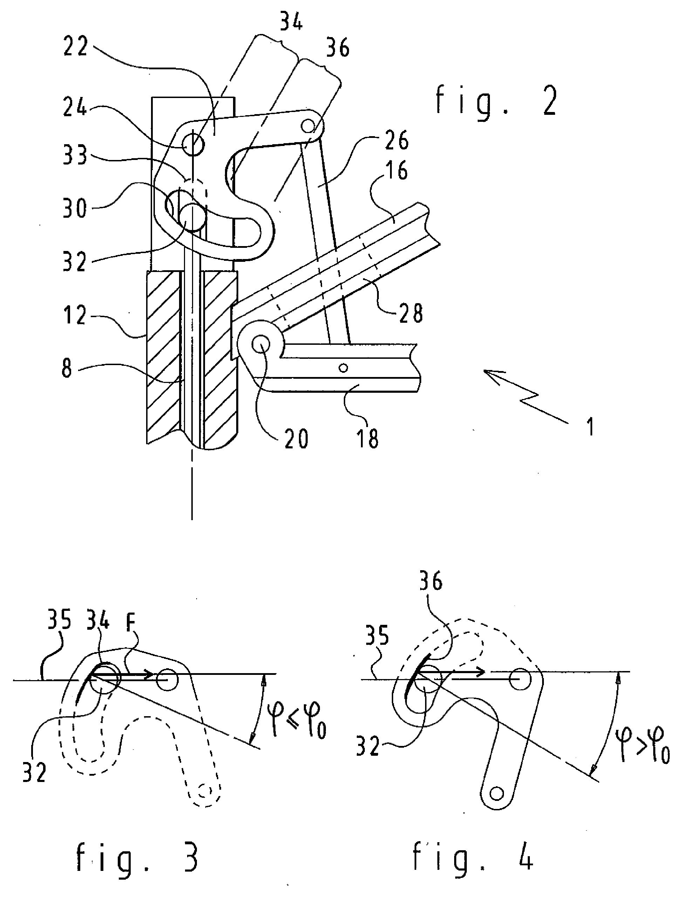

Aux figures 1 et 2, d'une part, et 5 et 6, d'autre part, sont représentées deux variantes d'exécution du moyen de couplage à friction selon l'invention.

L'Homme du Métier adaptera aisément la forme de la pièce de couplage 22, de la bielle 26 et du chemin 30, afin de moduler les paramètres de contrôle de l'outil de travail en fonction de l'angle d'ouverture de l'ensemble à poignées 4. De même, le positionnement des axes de pivotement de la bielle sur le deuxième élément de poignée 18 ou la pièce de couplage 22 sera adapté en fonction.

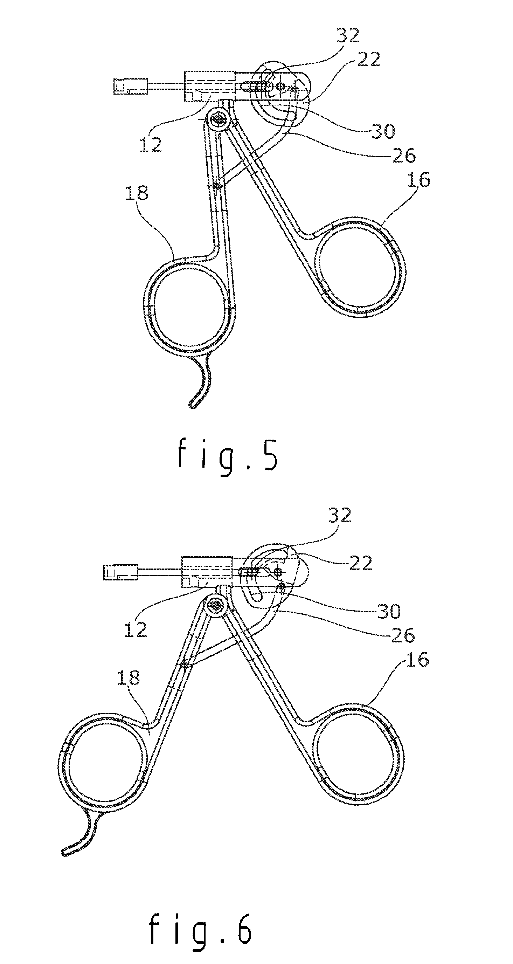

Ainsi, l'exemple d'exécution donné aux figures 5 et 6 est particulièrement préféré. Entre la position mors fermé (figure 5) et mors ouvert (figure 6), l'écartement entre les éléments de poignée passe de 25 ° à 51 ° pour une course totale de la tringlerie de 2mm.

On comprend bien l'avantage économique lié à la conception du moyen de couplage par friction selon l'invention, qui ne nécessite qu'une adaptation de la pièce de couplage et de la bielle en fonction des souhaits de contrôle des utilisateurs, tout en conservant à l'identique les autres éléments de l'ensemble à poignées.

Le chemin comprend une première partie 34 dans laquelle la goupille 32 se meut avec blocage lorsque la pièce de couplage 22 pivote et une deuxième partie 36 dans laquelle elle se meut sans blocage, en l'absence d'action de l'opérateur sur le deuxième élément de poignée 18.

L'intensité du frottement (notamment la valeur ϕ₀ du demi-angle au sommet du cône de frottement) de la goupille 32 sur le chemin 30 peut être sélectionné facilement par l'homme du métier en tribologie et notamment en fonction des matériaux utilisés, de leur état de surface, de leur lubrification, du profil de chaque partie du chemin, etc, pour obtenir une partie de chemin où la goupille est bloquée et une partie de chemin où la goupille n'est pas bloquée.

Les figures 3 et 4 illustrent schématiquement, à titre d'exemple, l'influence du profil sur la condition de blocage. Sur la figure 3, la goupille se trouve dans la partie de chemin 34. On a représenté l'angle ϕ entre la résultante F de l'action de la goupille 32 sur la partie de chemin 34, et la normale à la surface de contact entre la goupille 32 et la partie de chemin 34. Le mouvement de la goupille 32 suivant l'axe 35 est bloqué car ledit angle ϕ est inférieur à ϕ₀, et donc la résultante F se trouve à l'intérieur du cône de frottement. Au contraire, sur la figure 4, l'angle ϕ entre la résultante F de l'action de la goupille 32 sur la deuxième partie du chemin 36, et la normale à la surface de la goupille 32 et la deuxième partie 36, est supérieur à ϕ₀. La résultante F est alors en dehors du cône de frottement. Ceci traduit le fait que la goupille n'est pas bloquée mais peut glisser dans la partie de chemin 36.

Par commodité, on a représenté uniquement le contact entre la goupille 32 et le chemin sur le bord inférieur du chemin. Bien évidemment, du fait du parallélisme des bords du chemin, on conçoit que le fonctionnement en mode blocage ou non blocage serait similaire si le contact entre la goupille et le chemin se situait sur le bord supérieur du chemin.

De manière préférée, le blocage est souhaité lorsque l'organe de travail est en position fermée (mors jointifs ou rapprochés en prise), ce qui est la position de sécurité pour déplacer l'organe de travail dans le corps du patient. Dans le mode de réalisation représenté, ceci est obtenu lorsque le deuxième élément de poignée 18 est ramené vers le premier élément de poignée 16, ce qui correspond à la position naturelle des objets tels que des paires de ciseaux.

On pourrait toutefois faire le choix inverse.

Il est également possible de configurer le chemin et/ou la goupille pour qu'un frottement suffisant ait lieu dans toutes les positions des éléments de poignée.

## Revendications

1. Ensemble à poignées, notamment pour instrument chirurgical endoscopique, comprenant un corps tubulaire (12) destiné à recevoir une extrémité d'une tringlerie (8) mobile axialement dans ledit corps tubulaire, un premier élément de poignée (16) monté fixe audit corps tubulaire, un deuxième élément de poignée (18) monté de manière pivotante sur ledit corps tubulaire, et un moyen de couplage pour convertir un mouvement de pivotement du deuxième élément de poignée en un mouvement de translation axial de ladite tringlerie, **caractérisé en ce que** ledit moyen de couplage comprend une pièce de couplage (22) montée pivotante sur ledit corps tubulaire (12) et reliée par une bielle (26) au deuxième élément de poignée (18), ladite pièce de couplage comprenant un chemin (30) pour guider un mouvement axial de l'extrémité de la tringlerie, en configuration opérationnelle, au moins une partie dudit chemin et l'extrémité de la tringlerie étant configurés pour qu'un frottement suffisant existe et bloque les éléments de poignée en position lorsque ces éléments de poignée ne sont pas actionnés.

2. Ensemble à poignées selon la revendication 1, **caractérisé en ce que** le chemin et l'extrémité de la tringlerie sont configurés pour qu'un frottement bloquant les éléments de poignées existe sur l'ensemble dudit chemin.

3. Ensemble à poignées selon l'une des revendications 1 ou 2, **caractérisé en ce que** ledit chemin (30) est formé d'une rainure ou d'une fente.

4. Ensemble à poignées selon l'une des revendications 1 à 3, **caractérisé en ce qu'**au moins une partie (34) du chemin a un profil en spirale par rapport au pivot (24) de la pièce de couplage (22).

5. Instrument chirurgical endoscopique comprenant :
- un tube (2) ayant une extrémité proximale et une extrémité distale, une tringlerie (8) étant disposée dans ledit tube,
- un organe de travail (6) disposé à l'extrémité distale du tube (2) et étant fixé à une extrémité de la tringlerie (8), et
- un ensemble à poignées (4) selon l'une des revendications précédentes monté à l'extrémité proximale du tube (2).

6. Instrument chirurgical endoscopique selon la revendication 8, **caractérisé en ce** l'extrémité proximale de la tringlerie (8) est équipé d'une goupille (32) agencée pour se mouvoir sur le chemin (30) du moyen de couplage.
